# EUROPEAN PATENT SPECIFICATION

(11) **EP 0 703 977 B1**
(45) Date of publication and mention of the grant of the patent: **05.11.1997**
(21) Application number: 94916345.5
(22) Date of filing: 01.06.1994
(51) Int. Cl.: C12M 3/06, C12M 1/32

(54) **WELL INSERTS FOR USE IN TISSUE CULTURE**
EINSÄTZE ZUR VERWENDUNG BEI DER GEWEBEKULTUR
PIECES RAPPORTEES POUR PUITS UTILISEES EN CULTURE TISSULAIRE

(30) Priority: 01.06.1993 GB 9311276
(43) Date of publication of application: 03.04.1996
(73) Proprietor: WHATMAN plc, Kent ME16 OLS (GB)
(72) Inventor: PHILPOTT, Richard William, Whatman plc, Kent ME16 0LS (GB); JONES, Hywel, Whatman plc, Kent ME16 0LS (GB); MARCH, Elizabeth Jane, Whatman plc, Whatman House, Kent ME16 0LS (GB)
(74) Representative: Perkins, Sarah
(86) International application number: GB9401192
(87) International publication number: WO9428111

(56) References cited:
- EP-A- 0 239 697
- EP-A- 0 495 213
- COSTAR EUROPE PRODUCT INFORMATION BROCHURE November 1986 , BADHOEVEDORP, NL pages 1 - 12 'COSTAR GIVES MEMBRANE TECHNOLOGY NEW DIMENSIONS.'

## Description

This invention relates to well inserts intended primarily for use in conjunction with microtitre plates in cell and tissue culture.

Animal cell cultures are frequently grown in-vitro using a range of different types of container such as bottles or tubes made from either glass or plastic which may be treated for tissue culture applications. For more specialised biological, biochemical, histological or immunological procedures, animal cell cultures may be grown in small volumes of growth medium within containers referred to as tissue culture inserts: A typical example of this product is cited in U.S. Patent 4,963,490. The base of the tissue culture insert is composed of a thin porous membrane which may be either inorganic or polymeric, e.g. alumina or polycarbonate. The growth medium containing the animal cell culture is placed within the insert. This insert is then placed within an outer chamber also containing a set volume of growth medium, the cells are cultured on the upper surface of the porous membrane. One problem encountered with such an insert is that cells have a tendency to grow up the inner surface of the side wall of the insert and interfere with the monolayer of cells formed on the membrane.

For some techniques such as studies of cell to cell interactions, one type of cell culture can be grown on the upper membrane surface and another cell type can be grown on either the lower membrane surface or the bottom surface of the outer chamber; the two cultures being separated by the porous membrane (co-cultivation).

Where multiple numbers of tests are required a microtitre plate format is often used for cell growth. The standard format is a one-piece moulding providing an array of 96 wells arranged in 12 columns of 8 wells. The internal diameter of each well is typically 7mm, the depth varies between 10-12mm and the overall size of the plate is 125mm x 85mm.

The flat bottom walls of these wells are moulded integrally with the whole plate from a plastics material and are non-porous. These wells contain the growth medium, and animal cells are grown on the bottom walls of the wells.

The miniaturisation of tests which is possible with the use of these multiple-well microtitre plates is advantageous, in addition to reducing the bulk of the well array and the relative ease of transport of the array, in that:
(i) tests can be miniaturised to reduce the cost of expensive materials e.g. growth medium, additives and assay chemicals,
(ii) multiple numbers of wells can be manually dosed with liquid simultaneously,
(iii) automated equipment is available for rapid addition of liquids to individual wells either sequentially or simultaneously, and
(iv) automated equipment is available-for rapid analysis of test assays e.g. by UV spectrophotometry and fluorescence.

However, these microtitre plates, used alone, have some disadvantages:
(i) the lack of a porous growth support and barrier layer prevents their use to provide
   a) assays such as chemical transport studies or certain types of toxicology testing procedures (e.g. the Fluorescein leakage test), and
   b) growth of two different cell types within the same well for co-cultivation of cells for cell-to-cell interaction studies.
(ii) The majority of epithelial cells do not differentiate or polarize on non-porous supports.
(iii) Cells cannot be studied by electron microscopy.
(iv) Adhered cells cannot be transferred between wells.

In EP-A-0239697 an individual well of a tissue culture dish is shown. The well is generally cylindrical and has an individual membrane support assembly which is essentially tubular and has an outwardly extending flange at its upper, open end, which flange serves to position the support assembly in the well. The lower end of the support assembly is closed by a porous membrane.

The present invention provides an array of well insert elements for use with a microtitre plate, each well insert element comprising a transparent side wall of frusto-conical form having its base end closed by a porous inorganic or polymeric membrane, the periphery of which is sealed with respect to the side wall and having an outwardly projecting flange at its upper, larger diameter, end, the flanges of the well insert elements merging together to form an integral support flange for the array, and the array further having means for spacing the side walls of the elements uniformly from surrounding side walls of respective cells of the microtitre plate.

The support flange is arranged to support the array on a microtitre plate which provides an individual cell to receive a well insert element of the array. The array may conveniently be in the form of a strip providing, for example, a row of 8 insert elements in length and being a single insert element wide.

Preferably, the internal diameter of the base is in the range 3.0mm to 4.5mm. Ideally the diameter is substantially 3.2mm.

The invention also provides in combination a microtitre plate moulded from a plastics material and providing an array of individual wells which are arranged in rows and which are open at the top and closed at the bottom, a strip of well insert elements moulded from a plastics material and arranged to be suspended in respective wells of one of said rows, each of said insert elements being open at the top and having a frusto-conical side wall defining an insert well which is closed at its lower end by an inorganic or polymeric porous membrane the periphery of which is sealed with respect to said side wall, the upper ends of said frusto-conical side walls being formed integrally with a support flange which rests on the microtitre plate and which covers annular gaps formed between the side walls of the wells of the plate and the external surfaces of the side walls of the respective insert elements, and spacing means for spacing the side walls of the insert elements uniformly from the surrounding side walls of the respective wells of the plate.

The distance between the bottom of each well and the underside of the membrane of the insert element occupying the well is preferably in the range of 0.5mm to 3mm. Ideally the distance is substantially 1mm.

The invention will now be described in more detail with reference by way of example to the accompanying drawings in which:
Figure 1 shows in sectional elevation a well insert element according to the invention in place over a row of wells of a microtitre plate,
Figure 2 is a plan view corresponding to Figure 1 but omitting the lid, and
Figure 3 shows on a larger scale a single well of the insert element.

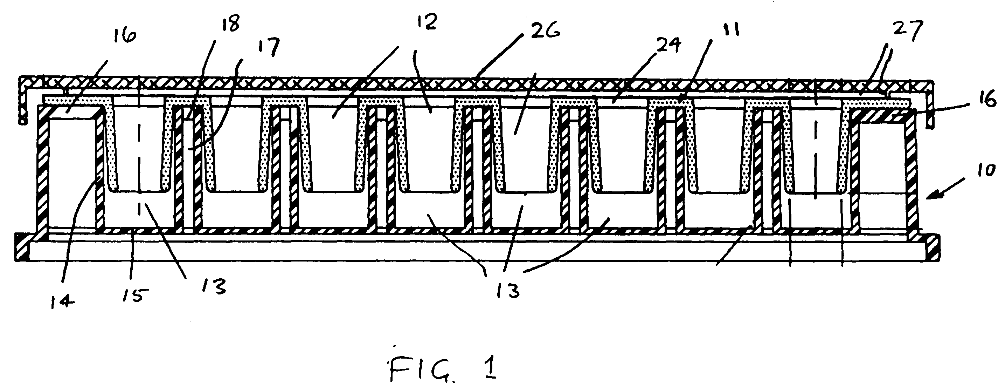

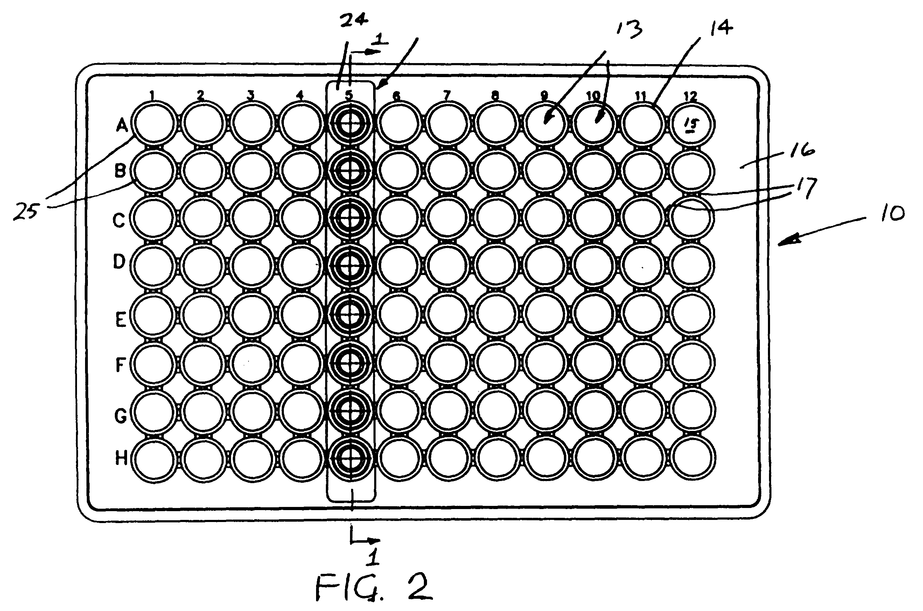

Referring to Figures 1 and 2 of the drawings, the microtitre plate 10 is an integral plastics moulding which is transparent in this instance and which provides an array of 96 wells arranged in twelve rows of eight wells. The well insert 11 has, arranged in a strip, eight insert elements 12 disposed in the individual wells 13 of one row of eight.

Each well 13 has a substantially cylindrical side wall 14 and a bottom wall 15. The top wall 16 of the plate 10 is continuous along the periphery of the plate but is discontinuous between the wells, the side walls 14 of adjoining wells being interconnected by vertical webs 17 which terminate at 18, short of the top edges of the side walls 14.

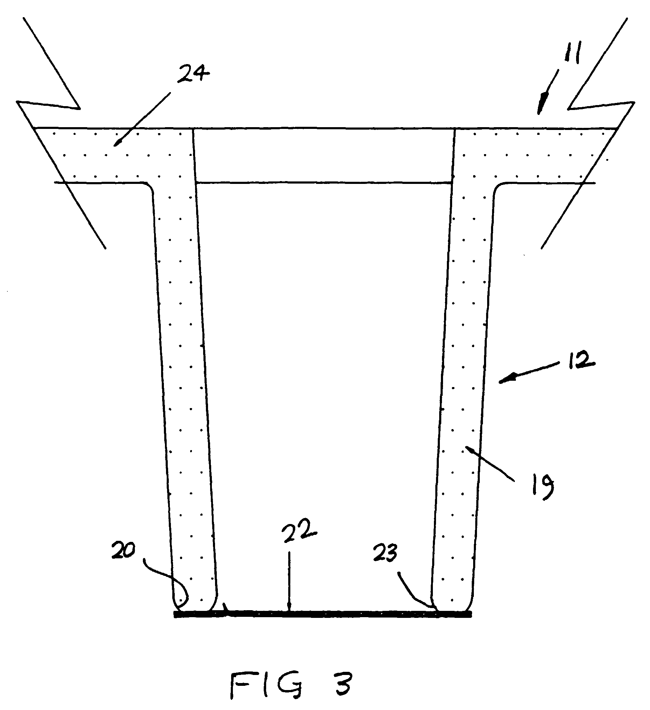

The well insert 11 (see also Figure 3) comprises a strip which is a transparent plastics moulding with the eight insert elements 12 forming the strip arranged so as to be centred in the respective wells 13 of a single row. Each well insert element has a frusto-conical side wall 19 of substantially uniform thickness except at its lower end 20, which is rounded. A porous membrane 22, made for example of alumina or polycarbonate, is sealed across the lower end of the side wall of the insert. An acute angle 23 is formed where the membrane joins the rounded end 20. An undercut region is thereby formed where the membrane meets the periphery of the side wall. The undercut region is effective in preventing cells growing up the inner surface of the side wall of the insert element beyond the upper surface of a monolayer of cells grown on the membrane.

The well insert elements are joined together at their upper ends by a flange member 24 extending along the length of the row of insert elements. When in use the flange member 24 rests on the tops 25 of the side walls of the wells, which project a small distance above the level of the top wall 16 of the plate 10. This arrangement prevents or reduces loss of liquid from the wells by evaporation and reduces the risk cf well-to-well interference in the tests being carried out. A lid 26 (not shown in Figure 3) for the plate 10 has a rib 27 on its underside which rests on the ends of the flange member 24.

Small projections (not shown) are formed on the underside of the flange member adjacent its opposite ends beyond the outermost insert elements and engage in the annular gap between the sidewalls of the endmost wells and their insert elements. These projections serve to centralise all of the insert elements of the strip in their respective wells.

In the illustrated construction the width of the flange member 24, which is one insert element wide, is small enough to allow a strip of insert elements to be located in each row of wells in the plate 10 with suitable clearances between adjoining strips to facilitate handling of the strips. With a conventional microtitre plate twelve separate strips of insert elements may therefore be used. Alternatively, the insert element may provide two or more rows of insert elements and in any desired form of array having regard to the spacing of the wells in the plate. In this way a plurality of separate strips or arrays of insert elements are provided for a single conventional microtitre plate. By providing a plurality of strips or arrays for use with a single microtitre plate, each strip or array may be used for a separate multiple test thereby making the organisation and management of the multiple tests easier and reducing the risk of error. Also, the automated handling of the individual strips or arrays is greatly simplified where the strips or arrays extend over a row of wells of the microtitre plate since then the strip or array may be grasped at the edges of the plate.

In a particular design of well insert strip according to the invention for use with one row of 8 wells of a standard 96 well microtitre plate in which the wells are centred at intervals of 9mm ± 0.5mm and each well is substantially cylindrical, having internal diameters of 6.5m ± 0.5mm at its top and base ends, the top of the well being 0.5mm wider than the base end, the strip has a total length of 79mm and a width of 8.8mm ± 0.1mm, the pitch spacing of the inserts corresponds to that of the wells, and each insert has a constant sidewall thickness of 0.8mm ± 0.lmm, an internal diameter of 4mm ± 0.1mm at the top and an internal diameter of 3.2mm ± 0.1mm at the base. The internal diameter of the insert must be in the range 3.00mm to 4.5mm so as to enable the base of the insert to be examined by optical microscopy. The meniscus produced by liquid in the well prevents proper use of a microscope in this manner when the internal diameter of the well is reduced below 3.0mm. The preferred range of distances between the underside of the membrane and the upper surface of the base of the well is 0.5mm to 3mm. Ideally, the distance is 1mm ± 0.1mm. Within this range of distances free diffusion of molecules from the lower well is enabled. This range of distances is also particularly suitable for co-culture where soluble mediators are able to freely diffuse between the two layers of cells which are physically separated.

In a typical use of the plate and insert strip 11 a predetermined volume of a growth liquid is placed in each well, the insert strip 11 is lowered into the wells 13 and a predetermined volume of a growth medium suitable seeded with cells is placed in each well of the well insert. Growth of the cells to confluence is observed, and examined, by optical microscopy if desired. The insert strip 11 can be removed to enable washing and subsequent treatment of the cell growth in the wells of the insert by reagents or other treatment can be carried out in all of the wells of the insert simultaneously. Additionally or alternatively, cell growth on the bottom wall of the wells 13 may be examined and treated.

As mentioned above, the bottom edge 20 of the side wall of the well insert to which the porous membrane is attached is designed to produce an acute angle between the insert wall and membrane, e.g. by use of a rounded profile. At the point of attachment of the membrane to the element wall, the resulting acute angle 23 discourages growth of cells up the side wall of the insert element, restricting cell growth to the membrane surface only. This is required to prevent interference with the layer of cells on the membrane surface. Continuation of growth from the membrane up the walls of the well can result in the confluent layer on the membrane being disturbed when cells slough from the walls.

The strip 11 may be manufactured in a moulded plastic polymer material such as polystyrene, polycarbonate, polypropylene or similar material. The polymer may be non-pigmented to give a visually clear material or may contain a pigment to reduce optical clarity. An example is a black pigment which may be used to reduce stray fluorescent light when using sensitive types of analytical detectors.

The length of the flange member is selected to be long enough to enable the strip to be manually removed easily from the microtitre plate, but short enough to enable the lid of the microtitre plate to be fitted over the well inserts. 79mm ± 0.25mm has been found to be a good length for these purposes.

In one example the well insert has a capacity of 75 microlitres for the addition of growth medium. Maintaining the internal diameter of the lower end of the side wall at a value greater than 3.0mm, allows the growth medium to be introduced within the well insert without an air-lock forming over the base of the well insert below the growth medium. Such an air-lock can be difficult to remove and tends to interfere with proper functioning of the device. A volume of 150 to 200 microlitres of growth medium is required in the well of the microtitre plate in the presence of the insert element. Maintaining the external diameter of the base of the well insert to less than 5.6mm prevents an air-lock forming on the underside of the well insert element, within the growth medium on addition of the well insert element to the microtitre well. Such an air-lock causes displacement of growth medium from the microtitre well, prevents proper functioning of the device and can also result in cross-contamination of growth medium within adjacent wells. These measures combined with the provision for a uniform gap between the side wall of the insert and the wall of the microtitre well allows proper venting of excess air from the well insert element and microtitre well.

## Claims

1. An array of well insert elements for use with a multi-well plate, each well insert element (12) comprising a transparent side wall (19) of frusto-conical form having its base end closed by a porous inorganic or polymeric membrane (22), the periphery of which is sealed with respect to the side wall and having an outwardly projecting flange at its upper, larger diameter, end, the flanges of the well insert elements merging together to form an integral support flange (24) for the array, and the array further having means for spacing the side walls (19) of the elements (12) uniformly from surrounding side walls (14) of respective cells of the multi-well plate.

2. An array as claimed in claim 1, wherein the array is in the form of a strip with a plurality of well insert elements (12) along its length and being a single insert element in width.

3. An array as claimed in claims 1 or 2, wherein the internal diameter of the lower end of the side wall (19) of each well insert element (12) is substantially 3.2mm.

4. An array as claimed in any one of claims 1 to 3, wherein said means for spacing the side wall (19) of the element (12) uniformly from a surrounding side wall (14) of a well of a multi-well plate comprises projections formed on the underside of the support flange (24).

5. An array as claimed in any one of claims 1 to 4, wherein the inner surface of the side wall (19) of the well insert element (12) forms an acute angle to the plane of the porous membrane (12) thereby providing an undercut region where the side wall (19) of the well insert element (12) joins the porous membrane (22).

6. In combination a multi-well plate (10) moulded from a plastics material and providing an array of individual wells (13) which are arranged in rows and which are open at the top and closed at the bottom, a strip (11) of well insert elements (12) moulded from a plastics material and arranged to be suspended in respective wells (13) of one of said rows, each of said well insert elements (12) being open at the top and having a frusto-conical side wall (19) defining a well which is closed at its lower end by an inorganic or polymeric porous membrane (22) the periphery of which is sealed with respect to said side wall (19), the upper ends of said frusto-conical side walls (19) being formed integrally with a support flange (24) which rests on the multi-well plate (10) and which covers annular gaps formed between the side walls (14) of the wells of the plate and the external surfaces of the side walls (19) of the respective well insert elements (12), and spacing means for spacing the side walls (19) of the well insert elements (12) uniformly from the surrounding side walls (14) of the respective wells of the plate (10).

7. The combination claimed in claim 6, wherein the distance between the bottom of each well (13) and the underside of the membrane (22) of the insert element (12) occupying the well is substantially 1mm.

8. The combination claimed in claim 6 or claim 7, wherein the internal diameter of the lower end of the side wall (19) of each of said well insert elements (12) is substantially 3.2mm.

9. The combination claimed in any one of claims 6 to 8, wherein said spacing means comprises projections formed integrally with the strip and engaging the side walls (14) of two or more of the wells (13) at the upper ends of said side walls.

## Patentansprüche

1. Eine Anordnung tiefer Probenschälchen-Einsatzelemente zur Verwendung mit einer Multi-Probenschälchenplatte, wobei jedes tiefe Probenschälchen-Einsatzelement (12) eine transparente Seitenwand (19) von kegelstumpfartiger Form aufweist, deren unteres Ende durch eine poröse, anorganische oder polymere Membran (22) verschlossen ist, deren Peripherie in bezug auf die Seitenwand abgedichtet ist und ab ihrem oberen Ende größeren Durchmessers einen nach außen ragenden Flansch besitzt, wobei sich die Flansche der tiefen Probenschälchen-Einsatzelemente vereinen, um einen integralen Trägerflansch (24) für die Anordnung zu bilden, und die Anordnung weiter Mittel für die einheitliche Abstandshaltung der Seitenwände (19) der Elemente (12) von umgebenden Seitenwänden (14) der betreffenden Zellen der Multi-Probenschälchenplatte aufweist.

2. Eine Anordnung wie in Anspruch 1 beansprucht, worin die Anordnung in der Form eines Streifens mit einer Vielheit tiefer Probenschälchen-Einsatzelemente (12) entlang seiner Länge ist, und in der Breite ein Einzeleinsatzelement verkörpert.

3. Eine Anordnung wie in Ansprüchen 1 oder 2 beansprucht, worin der Innendurchmesser des unteren Endes der Seitenwand (19) jedes tiefen Probenschälchen-Einsatzelements im wesentlichen 3, 2 mm ist.

4. Eine Anordnung wie in einem beliebigen der Ansprüche 1 bis 3 beansprucht, worin genanntes Mittel für die einheitliche Abstandshaltung der Seitenwand (19) des Elements (12) von einer umgebenden Seitenwand (14) einer Multi-Probenschälchenplatte Vorsprünge aufweist, die auf der Unterseite des Trägerflansches (24) geformt sind.

5. Eine Anordnung wie in einem beliebigen der Ansprüche 1 bis 4 beansprucht, worin die Innenfläche der Seitenwand (19) des tiefen Probenschälchen-Einsatzelements (12) einen spitzen Winkel zur Ebene der porösen Membran(22) bildet und dadurch einen Unterschnittbereich bereitstellt wo die sich Seitenwand (19) des tiefen Probenschälchen-Einsatzelements (12) mit der porösen Membran (22) verbindet.

6. In Kombination eine Multi-Probenschälchenplatte (10), die aus einem Kunststoffmaterial geformt ist und eine Anordnung individueller tiefer Probenschälchen (13) bereitstellt, die in Reihen angeordnet sind und die oben offen und unten geschlossen sind, ein Streifen (11) tiefer Probenschälchen-Einsatzelemente (12), die aus einem Kunststoffmaterial geformt sind und angeordnet sind, um in betreffende tiefe Probenschälchen (13) einer der genannten Reihen gehängt zu werden, jedes der genannten tiefen Probenschälchen-Einsatzelemente (12) oben offen ist und eine kegelstumpfartige Seitenwand (19) aufweist, die ein tiefes Probenschälchen definiert, das an seinem unteren Ende durch eine anorganische oder polymere poröse Membran (22) geschlossen ist, deren Peripherie in bezug auf die genannte Seitenwand (19) abgedichtet ist, wobei die oberen Enden genannter kegelstumpfartiger Seitenwände (19) integral mit einem Trägerflansch (24) geformt sind, der auf der Multi-Probenschälchenplatte (10) aufliegt und die ringförmige Lücken abdeckt, die zwischen den Seitenwänden (14) der tiefen Probenschälchen der Platte und den externen Oberflächen der Seitenwände (14) der betreffenden tiefen Probenschälchen-Einsatzelemente (12) geformt sind, und Abstandshaltungsmitteln zum einheitlichen Abstandhalten der Seitenwände (19) der tiefen Probenschälchen-Einsatzelemente (12) von den umgebenden Seitenwänden (14) der betreffenden tiefen Probenschälchen der Platte (10).

7. Die in Anspruch 6 beanspruchte Kombination, worin der Abstand zwischen der Basis jedes tiefen Probenschälchens (23) und der Unterseite der Membran (22) des Einsatzelements (12), welches das tiefe Probenschälchen einnimmt im wesentlichen 1 mm ist.

8. Die in Anspruch 6 oder Anspruch 7 beanspruchte Kombination, worin der Innendurchmesser des unteren Endes der Seitenwand (19) jedes der genannten tiefen Probenschälchen-Einsatzelemente (12) im wesentlichen 3,2 mm beträgt.

9. Die in einem beliebigen der Ansprüche 6 bis 8 beanspruchte Kombination, worin genanntes Abstandshaltungsmittel Vorsprünge aufweist, die integral mit dem Streifen geformt sind und mit den Seitenwänden (14) von zwei oder mehr der tiefen Probenschälchen (13) an den oberen Enden der genannten Seitenwände in Eingriff stehen.

## Revendications

1. Un ensemble d'inserts pour puit destiné à être utilisé en conjonction avec une plaque multi-puits, chaque insert pour puit (12) comprenant une paroi latérale transparente (19) en forme de base de cone, le fond de l'insert étant fermé grâce à une membrane poreuse inorganique ou en polymère (22) dont la périphérie est scellée à la paroi latérale, la paroi latérale comportant en haut de l'insert, à l'endroit où le diamètre est le plus large, un rebord orienté vers l'extérieur, les rebords des inserts pour puit se rejoignant pour former un rebord de support intégral (24) destiné à supporter l'ensemble, l'ensemble comprenant en outre un dispositif permettant de laisser un espacement uniforme entre les parois latérales (19) des inserts (12) et les parois latérales (14) des puits respectifs de la plaque multi-puits les entourant.

2. Un ensemble ainsi revendiqué à la revendication 1, dans lequel l'ensemble a la forme d'une bande comportant sur sa longueur plusieurs inserts pour puit (12), la largeur de la bande étant égale à celle d'un seul insert.

3. Un ensemble ainsi revendiqué à la revendication 1 ou à la revendication 2, dans lequel le diamètre interne de la partie inférieure de la paroi latérale (19) de chaque insert pour puit (12) est de l'ordre de 3,2mm.

4. Un ensemble ainsi revendiqué à l'une des revendications 1 à 3, dans lequel ledit dispositif permettant de laisser un espacement uniforme entre les parois latérales (19) de l'insert (12) et les parois latérales (14) du puit de la plaque multi-puits entourant l'insert comporte des saillies montées sur le dessous du rebord de support (24).

5. Un ensemble ainsi revendiqué à l'une des revendications 1 à 4, dans lequel la surface interne de la paroi latérale (19) de l'insert pour puit (12) forme un angle aigu avec le plan de la membrane poreuse (22), définissant ainsi une zone creuse où la paroi latérale (19) de l'insert pour puit (12) rejoint la membrane poreuse (22).

6. Un dispositif combinant une plaque multi-puits (10) moulée dans un matériau plastique et offrant un ensemble de puits individuels (13) disposés en rangées, ouverts en haut et fermés en bas, une bande (11) d'inserts pour puit (12) moulés dans un matériau plastique et disposés en sorte qu'ils puissent être suspendus à l'intérieur de chacun des puits respectifs (13) de l'une desdites rangées, chacun desdits inserts (12) étant ouvert en haut et comportant un paroi latérale en forme de base de cone (19) et définissant un puit qui est fermé au niveau de son extrémité inférieure par une membrane poreuse inorganique ou en polymère (22) dont la périphérie est scellée à ladite paroi latérale (19), les extrémités supérieures desdites parois latérales en forme de base de cone (19) fusionnant intégralement avec un rebord de support (24) qui repose sur la plaque multi-puits (10) et recouvre les espacements annulaires séparant les parois latérales (14) des puits de la plaque des surfaces externes des parois latérales (19) de chaque insert (12), et un dispositif d'espacement permettant de laisser un espacement uniforme entre les parois latérales (19) des inserts (12) et les parois latérales (14) des puits de la plaque (10) les entourant.

7. La combinaison ainsi revendiquée à la revendication 6, dans laquelle la distance entre le fond de chaque puit (13) et le dessous de la membrane (22) de l'insert (12) placé dans le puit est de l'ordre de 1mm.

8. La combinaison ainsi revendiquée à la revendication 6 ou à la revendication 7, dans laquelle le diamètre interne de la partie inférieure de la paroi latérale (19) de chaque insert pour puit (12) est de l'ordre de 3,2mm.

9. La combinaison ainsi revendiquée à l'une des revendications 6 à 8, dans laquelle ledit dispositif d'espacement comporte des saillies fusionnant intégralement avec la bande et s'emboîtant dans les parois latérales (14) d'au moins deux des puits (13) au niveau des extrémités supérieures desdites parois latérales.
